# EUROPEAN PATENT SPECIFICATION

(11) **EP 3 280 395 B1**
(45) Date of publication and mention of the grant of the patent: **27.03.2019**
(21) Application number: 16726669.1
(22) Date of filing: 07.04.2016
(51) Int. Cl.: A61K 9/107, A61K 47/14, A61K 47/32, A61K 47/44, A61K 31/202, A61K 31/201, A61K 31/231, A61K 31/232

(54) **OPHTHALMIC COMPOSITIONS BASED ON POLYUNSATURATED FATTY ACIDS AND TRIACYLGLYCEROLS**
OPHTHALMISCHE ZUSAMMENSETZUNGEN AUF BASIS VON MEHRFACH UNGESÄTTIGTEN FETTSÄUREN UND TRIACYLGLYCERINEN
COMPOSITIONS OPHTALMIQUES À BASE D'ACIDES GRAS POLYINSATURÉS ET DE TRIACYLGLYCÉROLS

(30) Priority: 08.04.2015 IT UB20150343
(43) Date of publication of application: 14.02.2018
(73) Proprietor: For Health Pharma S.r.l., 95127 Catania (CT) (IT)
(72) Inventor: MANGIAFICO, Sebastiano, 95127 Catania (CT) (IT)
(74) Representative: Banchetti, Marina
(86) International application number: PCT/IT2016/000086
(87) International publication number: WO 2016/162894

(56) References cited:
- WO-A1-2014/035450
- WO-A2-2010/106571
- US-A1- 2006 251 685

## Description

### Field of the invention

The present invention concerns ophthalmic preparations based on polyunsaturated fatty acids and triacylglycerols. More specifically, the invention relates to preparations for topic ophthalmic use for the prevention and treatment of corneal-conjunctival pathologies having an inflammatory component, which preparations contain, as active ingredients, omega-3 and/or omega-6 polyunsaturated fatty acids in an oily vehicle dispersed in an aqueous gel, and which are stable to storage at room temperature.

### Background of the invention

It is known that the preocular tear film is a complex liquid structure which coats the exposed surface of the eyeball, as well as the bulbar and the palpebral conjunctiva. This structure results from the cooperation of a solid layer, formed by the complex of corneal epithelium and glycocalyx (i.e., the glycoprotein coating of the epithelial cells, consisting of their secretions), with a liquid layer, which is more properly the lacrimal film. The solid layer has the function of allowing the adhesion of the liquid part of the lacrimal film to the ocular surface, while the liquid layer is formed in turn by three overlapping layers, of different constitution: a mucous layer, an aqueous layer and a lipid layer.

The internal mucous layer of the fluid tear film consists of a mixture of viscoelastic hydrated glycoproteins (mucin), which adhere to said solid layer and form a hydrophilic surface. The aqueous layer is the intermediate portion of the tear film, which spreads over said hydrophilic surface and is made essentially of water, organic and inorganic salts, sugars, proteins, enzymes and other biopolymers of a complex structure, such as the mucins themselves. The substances in solution in this layer have structural, osmotic, buffering and nourishing functions, and protect the lacrimal film from the tissues of the ocular surface. Finally, the thin external lipid layer is formed by waxes, fatty acids and cholesterol esters, and serves to stabilize the tear film, by controlling the water loss due to evaporation.

As a result of abnormalities or imbalances of one or more of the layers described above, the condition known as *dry eye syndrome* or *dry eye* may occur, which is a chronic disorder affecting mainly elderly people, mostly women. It is a multifactorial pathology characterized by modifications in the qualitative and quantitative composition of the tear film, which result in foreign body sensation, symptoms of irritation (discomfort), disturbances of vision and tear film instability, with potential impairment of the ocular surface. Dry eye is accompanied by hyperosmolarity of the tear film (with levels which may reach 330-340 mOsm/l, against a normal baseline value of about 300 mOsm/l), and often also by inflammatory phenomena, which extend from the ocular surface to the lacrimal gland and Meibomian glands.

Dry eye syndrome has in general a significant impact on the quality of life of patients and important social costs, due to the chronicity of symptoms, the reduction of visual ability in work and in daily living activities (reading, watching TV, driving), the need to have frequent recourse to examination by an ophthalmologist and to medical therapies. Given the progressive increase in the average age of the human population, such disease is going to have an ever increasing importance in the future.

The therapies for dry eye syndrome are mainly focused on restoring the tear film, by means of slow release ocular inserts to be inserted in the conjunctival sac and, more frequently, with liquid ophthalmic preparations, generally known as "artificial tears", to be instilled in drops in order to replace or integrate the natural tears production. In the simplest case such preparations have a moistening action only, as they consist of physiological saline solutions, neutral and isotonic with the tear fluid, based on sodium chloride only, or on balanced mixtures of various electrolytes. In other cases, in order to overcome the drawback of the reduced retention in the conjunctival sac and to lubricate the tissues and more effectively prevent the formation of dry areas in the corneal epithelium, the tear substitute formulations are enriched with high molecular weight components having functions of viscosifying agents. Such components are normally water-soluble polymers of a synthetic, semisynthetic or natural origin, many of which, such as hyaluronic acid and cellulose derivatives, have reached a widespread diffusion for the concerned use.

It is to be noted that in the case of alterations or insufficiency of the outer lipid layer of the tear film, the continuous exposure of the film to external environment may cause evaporation of the aqueous component, exposure of the ocular surface to infective agents and consequent inflammation of the same ocular surface. These mechanisms underlie the clinical signs and symptoms of dry eye syndrome, but can also occur in normal subjects exposed to environments with low humidity and flowing air.

Owing to the inflammatory component which is generally present in dry eye syndrome, compounds such as polyunsaturated fatty acids, in particular of the type omega-3 and omega-6, are quite interesting as candidates for inclusion in tear substitute preparations. As it is known, polyunsaturated fatty acids or PUFAs are carboxylic compounds with aliphatic chain having two or more double bonds in the chain, which are characterized by the position of the first double bond starting from the terminal carbon atom of the chain (ω position). Among the polyunsaturated fatty acids, omega-3 (or PUFA n-3) and omega-6 (or PUFA n-6) represent a group of essential fatty acids, indispensable for a proper functioning of the body. Examples of omega-3 fatty acids include α-linolenic acid (C18:3, n-3; ALA), eicosapentaenoic acid (C20:5 n-3; EPA) and docosahexaenoic acid (C22:6, n-3; DHA); examples of omega-6 fatty acids include linoleic acid (C18:2, n-6; LA) and γ-linolenic acid (C18:3, n-6; GLA).

Nutraceutical supplements based on polyunsaturated fatty acids, rich in omega-3 fatty acids, taken from fish oils (in particular, salmon and herring oils) and in omega-6 fatty acids, mostly of a vegetal origin (blackcurrant, borage) are widely employed in view of their beneficial activity on the cardiovascular, immune and nervous system and also, specifically, for use as supplements in the treatment of dry eye. It should be noted that, also in case of ophthalmic indications, these products are for oral administration.

It is evident that products for topical use in eye drops involve significant advantages for the treatment of dry eye, at least in view of the greater tolerability of a topical ophthalmic product in comparison with a product for oral administration, the use of which involves the gastrointestinal tract. Manufacture and marketing of such products, however, are conditioned by the difficulties encountered in formulating fatty acids of the type of PUFAs, especially for the poor water solubility and poor chemical stability of these active ingredients.

Polyunsaturated fatty acids, in fact, are highly lipophilic and poorly water-soluble molecules, and their formulation in commercial preparations for topical ophthalmic use involves considerable difficulties. Specifically, the most notable of these difficulties arise from the poor solubility of these compounds in water and from their reduced stability to oxidation phenomena.

The difficulties related to poor water solubility of PUFAs have been addressed by the prior art by using the emulsions technology, or by dispersing the oil in a viscous aqueous media, in particular a hydrogel.

Lipid emulsions, used since long time for parenteral applications, have been studied in order to formulate several lipophilic active ingredients and enhance their ocular bioavailability. As is well known, emulsions are dispersed systems formed by two immiscible liquid phases, prepared by mechanical stirring. Given the difference of attractive interaction between the different molecules of the two liquid phases, an interfacial tension is generated in each point where the two liquids are in contact, and due to said tension the two liquid phases tend to separate from each other, in order to minimize the contact surface. The interfacial tension may be significantly reduced by adding amphiphilic molecules or surface-active agents soluble in at least one of the two phases of the emulsion. Therefore, the addition of a suitable surface-active agent can avoid the separation of the aqueous phase from the oil phase, or at least it can slow down its progress.

Lipid emulsions for use as medicinal preparations, in particular pharmaceutical compositions consisting of emulsions of the oil-in-water type for use as a vehicle of lipophilic active ingredients, are disclosed in EP 0391369 (on behalf of Yissum Resarch Development Company of the Hebrew University of Jerusalem, inventors B. Simon and L. Menashe). Such compositions comprise an oily vehicle consisting of medium-chain triglycerides (MCT), optionally combined with a vegetable oil, such as, e.g., soybean oil, together with phospholipids (e.g., lecithin, or soy phospholipids) and surfactants, in particular non-ionic surfactants (such as, e.g., polysorbate 80 or Tween 80) and ionic surfactants (in particular, cholic and deoxycholic acid).

The application of the above technology to formulations containing PUFAs as active ingredients gave unsatisfactory results, as an emulsion based on omega-3 and omega-6 fatty acids obtained in this way is rather unstable from the physical point of view, and tends to evolve towards phase separation. In addition, and more critically, this emulsion is unstable from the chemical point of view, as the title of the two active ingredients is reduced in a considerable manner after only one month of refrigerated storage (at 4°C temperature), most likely as a result of oxidation.

More recently, the international patent application publ. No. WO2006/007510 (on behalf of Scheppens Eye Research and Johnson & Johnson Vision Care, Inc., inventors R. Dana et al.) described topical ophthalmic compositions based on fatty acids omega-6 and omega-3 as active ingredients, starting from the recognized anti-inflammatory efficacy of such agents and from the finding that an oral administration of the same, practiced for a long time, may be poorly tolerated or undesired.

In the preparations exemplified in such document, omega-3 and omega-6 fatty acids are directly emulsified with suitable surfactants, such as e.g. polyethoxylated sorbitan fatty acids esters (namely, polisorbates such as "Tween") and polyethoxylated methyl glucosides (such as "Glucam"). In the preparation process disclosed a first surfactant is added to a buffered saline solution and the mixture is kept under stirring, at room temperature, for a time sufficient to obtain a clear solution; then a second surfactant is added and subsequently, after a further period of mixing, the fatty acid (or fatty acids, in the event these are more than one) is/are added very slowly. Finally, one drop of vitamin E is added (with antioxidant function), and the emulsion is kept under stirring for some hours more.

It has however been ascertained, through further experimentation, that formulations based on EPA, DHA and GLA, obtained in emulsion according to the teachings of WO 2006/007510 suffer from oxidation problems similar to those already observed with the formulations in lipid emulsion described above.

The precarious physical stability of the emulsions, achieved only with the use of considerable quantities of surfactant (potentially toxic to the corneal surface) and, especially, the chemical instability of the polyunsaturated fatty acids of interest, have been addressed in the international patent application publ. No. WO2010/106571 (Medivis srl). This document proposes to disperse the oil-in-water emulsion containing the PUFAs in a viscous aqueous medium, in particular an aqueous hydrogel formed from hydrophilic polymers, which are able to entrap and suspend strongly hydrophobic active ingredients, without having to resort to the emulsion technology.

The foregoing allows to solve the problem of the physical instability of the compositions based on PUFAs for topical ophthalmic use. As regards the stabilization to oxidation, resort is made to the use of antioxidants, in particular α-tocopherol or vitamin E acetate. More precisely, in the formulations according to WO2010/106571 Vitamin E may be represented by any of the eight components of its family, i.e. α-, β-, γ-, δ-tocopherol and α-, β-, γ-, δ-tocotrienol, but α-tocopherol is preferred for its wider commercial availability and its greater activity. Among the relevant esters, in addition to succinate and esters with long-chain acids, the cheapest and most popular ester product is acetate. Therefore, the preferred anti-oxidant for the formulations of the cited document is α-tocopheryl-acetate, which, compared to the corresponding α-tocopherol, is more tolerable for a topical ocular administration. On the ocular surface, α-tocopheryl-acetate provides α-tocopherol (vitamin E) by hydrolysis.

Also the international patent application published under **No.** WO2014/ 035450 (Bausch & Lomb Inc.) describes topical ophthalmic preparations based on omega-3 fatty acids formulated in an aqueous gel vehicle. In this case, the gel is made from a weakly cross-linked carboxylic polymer (in particular, polycarbophil, known with the trade name NOVEON AA) having an appropriate concentration of components in the form of ionic salts, such as to show a viscosity that is reduced once the product is instilled in the eye and is in contact with the tear film, thus improving the ocular tolerability of the product.

It should be noted that the cited patent application takes into consideration the problem of physical stability of the composition in gel (by remarking that, once vigorously shaken before use, the described preparation maintains in suspension the oily fraction for at least two weeks), but not the problem of its chemical stability. The formulations described in WO2014/035450 may contain additional ingredients such as surfactants or emulsifiers, tonicity regulators (osmotizing agents), buffers, preservatives, co-solvents and viscosifying agents, but no mention is made in the document of any agents with specific antioxidant functions. In particular, vitamin E is mentioned as a possible optional ingredient together with other vitamins, including vitamins A, C, B₆, B₁₂ etc., no hint being made to any physical stability tests, much less to chemical stability tests, on the preparation.

Contrary to the last two of the above mentioned prior art documents, the patent application US 2006/0251685 (Yu Zhi-Jian et al.) discloses ophthalmic formulations based on omega-3 fatty acids in oil emulsion which are based on the conventional use of significant amounts of surfactants. The described preparations contain oil-globules that include an omega-3 fatty acid, dispersed in an aqueous vehicle, which may optionally contain a gelling polymer. This system is emulsified thanks to the presence of amounts of surfactants that are such as to confer to the oily mixture the ability to auto-emulsify. Accordingly, the oil globules which are formed in the preparation have very fine dimensions, in any case below a tenth of a micron.

### Summary of the invention

In the frame of the studies that led to the present invention it was considered that if on one hand the presence of an antioxidant such as α-tocopherol may minimize the formation of oxidation products in a preparation based on PUFAs as active ingredient, on the other hand tocopherol may also act as a pro-oxidant (Rietjens I.M.C.M. et al., the pro-oxidant chemistry of the natural antioxidants vitamin C, vitamin E, carotenoids and flavonoids, Environ. Toxicol. Pharmacol., 2002, 11 , 321-333).

Such antioxidant or pro-oxidant effect is strongly influenced by the agent concentration, the temperature and the physical system made up by the preparation in question. For this reason, in the preparations based on PUFAs stabilized with vitamin E it is necessary to dose vitamin E in such a manner that its concentration is not excessive, which would result in a pro-oxidant effect. At the same time said concentration should not be too low, in which case the agent would be consumed in a short time and the system would be left, at a certain point, without any antioxidant defense.

Some studies preliminary to the present invention have shown that the amount of vitamin E required to obtain preparations based on PUFAs which are stable for a time sufficiently long to allow the commercialization (i. e., having a shelf life longer than 18 months) coincides, unfortunately, with the pro-oxidant amount. For this reason, in preparations that contain α-tocopherol as antioxidant, as it also happens in many other formulations with other antioxidants, it is not possible to actually implement the conditions of chemical stability suitable for the omega-3 and -6 PUFAs.

In the formulations described as preferred in the Medivis patent application (WO2010/106571), the use of vitamin E acetate mixed with omega-3 and -6 PUFAs guarantees over time, thanks to the balance *vitamin E acetate* ↔ *vitamin E* + *acetate,* a constant, not pro-oxidant, concentration of α-tocopherol. With the use of the acetate derivative of vitamin E in the unique system described in the mentioned document, the antioxidant, long-lasting conditions, have been realized, which are necessary for a formulation based on omega-3 and -6 PUFAs, chemically stable for at least a certain period of time. To this regard it should be underlined that in the formulation of the cited patent application, in the absence of vitamin E acetate, or in the presence of α-tocopherol alone, the omega-3 and -6 fatty acids are chemically unstable. The corresponding experimental data are presented below in the experimental part of this description.

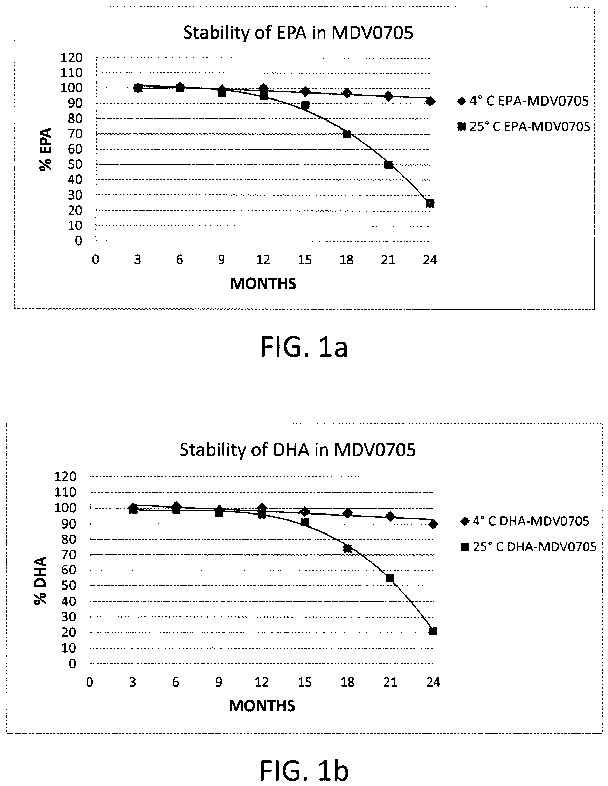

According to the studies carried out on the ophthalmic formulations with omega-3 and omega-6 fatty acids described in the patent application WO2010/106571, the preparations are stable when stored at room temperature for 12 months. The stability study reported in the document shows that at the temperatures of 4°C and 25°C the PUFAs degrade weakly for 12 months with a linear trend. However, the same stability study prolonged up to 24 months, reported in detail in the experimental section of the present description, still showed a linear trend for the formulations stored at 4°C, but showed a polynomial trend, indicative of a rapid degradation, for the formulations stored at 25°C (**Figures 1a** and **1b** discussed further below).

Therefore, the preparations based on PUFAs of the aforementioned prior art are not suitable to be stored at room temperature for at least 18 months, as required for a marketing authorization, but they have to be marketed under the condition of being stored in a refrigerated environment.

It is evident that the possibility of having available a topical ophthalmic composition based on PUFAs that is not only well-tolerated by the patients but also stable for long periods of time at room temperature, under conditions of non-refrigerated storage, would have the advantage of a considerable reduction of the management costs, as well better marketing opportunities, in particular by means of counter displays.

It has now been found that it is possible to obtain ophthalmic formulations based on PUFAs surprisingly stable in time, both physically and chemically, by using as a vehicle for the oily part of a formulation in hydrogel similar to the preparations of WO2010/106571 oily means, both vegetable and synthetic, formed by medium-chain triglycerides (MCT) or, possibly, also containing long chain triglycerides (LCT), provided that they are liquid at room temperature, in place of vitamin E acetate. Specifically, the stability of the proposed preparations according to the invention turned out to be greater than the stability achievable before with the use of antioxidants such as vitamin E and vitamin E acetate.

The object of the present invention is therefore a formulation in a stable aqueous gel, containing PUFAs as active ingredients, preferably in the form of alkyl esters or triglycerides, in which these PUFAs (omega 3 and/or omega-6) are dissolved in a vegetable or synthetic oil, in a concentration which may range from 5% to 85% by weight, and in which vitamin E or its esters with antioxidant activity are not present, nor surfactants in a total amount of more than 0.10% by weight are present.

The mixture of oil and polyunsaturated fatty acids forming the oily phase of the preparation according to the invention is dispersed in an aqueous hydrogel formed from one or more hydrophilic polymers suitable for ophthalmic use, for instance in a gel made from carbopol or another viscosifying or thickening polymer which can hold the oily phase dispersed in a stable manner. Limited quantities of one or more polymeric emulsifying agents and other water-soluble excipients are preferably added to the aqueous hydrogel phase, said excipients being selected from those normally used in products for topical ophthalmic administration.

### Detailed description of the invention

The present invention, thus, specifically provides a topical ophthalmic preparation indicated for the prevention and treatment of ophthalmic pathologies with an inflammatory component, in particular inflammatory kerato-conjunctivites and dry eye syndrome, containing, as active ingredients, one or more polyunsaturated fatty acids selected from the group consisting of: omega-3 polyunsaturated fatty acids and omega-6 polyunsaturated fatty acids, said polyunsaturated fatty acids having an aliphatic chain of from 16 to 24 carbon atoms, and pharmaceutically acceptable derivatives thereof selected from their esters with C₁-C₄ alkyl groups and their triglycerides, in solution in an oily vehicle,
said oily vehicle consisting of a vegetable or synthetic oil selected from the group consisting of: medium chain triglycerides (MCT), long chain triglycerides (LCT) liquid at room temperature, vegetable oils and their mixtures, and not containing vitamin E or pharmaceutically acceptable esters thereof,
the said solution being dispersed in form of oil globules in a hydrogel based on an aqueous vehicle containing one or more gelling polymers,
characterised in that said oil globules have an average diameter of 1-10 µm, and in that said preparation contains an overall amount of not more than 0.10% by weight of surfactants and emulsifying agents, and is substantially stable at room temperature, having a remaining content of undegraded active ingredients of not less than 90% by weight of the starting content after 18 months from the date of manufacture, when stored at 25°C.

In the preparations according to the invention, the mentioned one or more polyunsaturated fatty acids are generally dissolved in said oily vehicle in an overall concentration of from 5% to 85% by weight, preferably from 10% to 60%, and even more preferably from 15% to 50% by weight.

According to some specific embodiments of the invention, the polyunsaturated omega-3 and omega-6 fatty acids used in the formulation have aliphatic chains of from 18 to 22 carbon atoms, with three, five or six double bonds distributed along the chain.

According to some preferred embodiments of the invention, the said oil globules of the dispersed phase in the gel have a mean diameter between 1 and 5 µm, and preferably the average diameter of the globules of dispersed oil phase is from 2 to 4 µm.

Still preferably, the surfactants and emulsifying agents are present in the formulation according to the invention in an overall amount of not more than 0.010% by weight.

The formulation of the invention can also comprise one or more polymeric emulsifying agents, selected, for instance, from high molecular weight crosslinked polyacrylates (such as the product known under the trade name of "Pemulen", i.e. crosslinked polymer acrylates/alkyl acrylates C₁₀-C₃₀), poloxamers (polyoxyethylene-polyoxypropylene block copolymers such as the products known under the trade name "Pluronic") and D-alpha-tocopheryl polyethylene glycol succinate, known by the abbreviated name of TPGS.

In particular, the formulations according to the invention may contain as active ingredients eicosapentaenoic acid (EPA) or a linear or branched C₁-C₄ alkyl ester group or its triglyceride, and/or docosahexaenoic acid (DHA) or a linear or branched C₁-C₄ alkyl ester or its triglyceride. As the omega-6 polyunsaturated fatty acid, the above formulations may contain α-linolenic acid (GLA) or its linear or branched C₁-C₄ alkyl ester, or its triglyceride.

The preferred formulations for the preparations of the invention, in particular, contain as active ingredients EPA, DHA and optionally GLA, or their ethyl esters.

As is known, the PUFAs of the omega-3 family (such as EPA and DHA) are easily available in high concentrations in fish oils. Suitable sources may be selected from salmon oil, mackerel oil, oily fish oil, krill oil and mixtures thereof. The fatty acids of the omega-3 family are normally contained in such oils in percentages varying between 40 to 50%.

The sources of polyunsaturated fatty acids enriched in acids of the omega-6 family (such as GLA) may in turn be selected from the vegetable oils, in which lower levels of omega-3 are also present. Among the vegetable oils most used for this purpose are flaxseed oil, borage oil, wheat germ oil, hemp oil, olive oil, peanut oil, blackcurrant oil and soybean oil.

The pharmaceutical use of PUFAs, and in particular EPA, GLA and DHA, requires raw materials with a high degree of purity. Accordingly, they are separated from the mixture of fatty acids of fish and/or vegetable oils to reach a purity of 90% and 70%, respectively for EPA and GLA. The processes practiced today include extraction, molecular distillation and crystallization at low temperatures.

In the formulations of EPA and GLA or EPA, DHA and GLA, which are preferred according to the invention, Pemulen TR-1 is preferably used, at a concentration of from 0.001% to 0.15% of the total formulation, the preferred concentration being in the range of 0.01% and 0.10% by weight.

The oily vehicle to which the PUFAs are mixed to obtain the preparations of the invention consists of triacylglycerols (triglycerides), and preferably of synthetic or natural MCTs, such as coconut oil, or of vegetable oils containing predominantly LCTs, normally along with smaller amounts of MCTs.

According to official pharmacopoeias MCTs consist of mixtures of triglycerides of fatty acids with chains of 6-12 carbon atoms. The vegetable oil that contains the highest proportion of MCTs in its composition is coconut oil, while and high amounts of MCT are also present in palm oil (palm kernel oil). Examples of MCT products on the market, already used in the ophthalmic field are Miglyol® (Dynamit Nobel, SE) and TCM® (Societé des Oleagineux, FR).

With the exclusion of the two vegetable oils mentioned above, the majority of the other vegetable oils primarily contain LCTs, i.e. triglycerides of long chain fatty acids with from 13 to 21 carbon atoms. For instance, olive oil contains mostly triglycerides of oleic acid (C18: 1), linoleic acid (C18: 2 and palmitic acid (C16: 0).

As is known, both the triglycerides of vegetable origin and MCTs are widely used in ophthalmic products, to the point of being present as such and in the total absence of water in various formulations. For instance, cyclosporin has been formulated at 2% in olive oil (Diaz-Llopis, M. & Menezo, JL Penetration of 2% cyclosporin eyedrops into human aqueous humor. Br. J. Ophthalmol. 73, 600-603 (1989)), and in other vegetable oils (Lallemand, F., Felt-Baeyens, O., Besseghir, K., Behar-Cohen, F. & Gurny, R. Cyclosporine A delivery to the eye: to Pharmaceutical challenge. Eur. J . Pharm. Biopharm. 56, 307-318 (2003)). Similarly, azithromycin for ophthalmic use is commercially available not only in an aqueous formulation, but also in an exclusively oily preparation carried in MCT (European patent EP 1377316**,** Laboratoires Théa).

According to the present invention, in order to increase the chemical stability of PUFAs by protecting them from oxidation without having to resort to antioxidants based on vitamin E, some triglycerides have been used in the formulation of the oily phase, with the aim of isolating the PUFAs from the oxygen dissolved in water. In practice, the same system on which conservation of foods in oil is based has been used is to achieve a homogeneous solution of PUFAs in oil, and the obtained oily mixture was dispersed in an aqueous medium in gel for ophthalmic use. Indeed, It may be assumed that inhibition of oxidation can take place due to the fact that, in the oily phase, the PUFAs migrate and are hidden inside the droplet made by the triglycerides. Actually, at least as regards the MCTs, it has already been shown that they have a polarity sufficient to have a slight solubility in water (Galante, JH, & Tenor, RR, 2005, Medium-chain triglycerides. In CC Akoh (Ed.), Handbook of functional lipids, pp. 177-183, Boca Raton: CRC Press). The above would lead to an external surface in contact with water formed by the medium chain triglyceride, which acts as an insulator from oxygen and from all the radical species present in the aqueous phase (Food Chemistry, 2014, 152, 378-385).

According to some specific embodiments of the invention, therefore, the vegetable oils which may be used as vehicles in the oily phase of the product include coconut oil, castor oil, soybean oil, olive oil, corn oil, oil of sunflower seeds (sunflower oil), sesame oil and their mixtures.

In a preparation according to the invention containing EPA and DHA or the ethyl esters thereof as the active ingredients, the concentrations of EPA and DHA are each, preferably, from 0.001% to 1.20% by weight on the overall weight of the preparation, and in particular they are in the range from 0.01% to 0.50% by weight, and particularly preferred is the range from 0.01% to 0.15%. The concentration of the oily vehicle is in turn from 0.005% to 2% by weight, and in particular it is in the range of 0.10% to 0.60% by weight, the remainder being formed by said hydrogel.

The gelling polymers proposed for the formulation in hydrogel according to the invention are, preferably, products already used in the common medical practice, in particular polymers used as components of tear substitutes and having a mucoadhesive activity. These components are selected from components which not only do not alter the qualitative composition of the tear film, but may have an adjuvant activity to the anti-inflammatory of the polyunsaturated fatty acids. Among the gelling polymers that may be used in the proposed composition, also in combination with each other, the following are to be preferably considered: carboxyvinyl polymers (known as carbopol or carbomer), hyaluronic acid and its salts with alkali metals or alkaline earth metals, esters and ethers of cellulose (such as hydroxypropyl cellulose, hydroxypropylmethyl-cellulose, etc.), xanthan gum, alginic acid and alginates, and the gellans. Other gelling polymers used in the formulation of artificial tears suitable to prevent excessive evaporation of the aqueous lachrymal layer may also be used for the purpose of the invention.

According to some preferred embodiments of the invention, the gelling agent is carbopol polymer, optionally in combination with hyaluronic acid.

The preparations proposed according to the invention may also comprise, as is customary, pH regulators (such as for instance phosphate buffer, borate buffer and citrate buffer), sequestering agents such as EDTA, and osmotizing agents (regulators of tonicity), chosen from those currently used in pharmaceutical technology. In the preferred formulations of the invention, glycerol and trehalose are used as osmotizing agents in such quantity as to obtain a slightly hyposmotical formulation. The slight hypotonicity is a useful feature to counter the functional and anatomical suffering of the epithelia of the ocular surface, as is confirmed by the fact that in the cases of hyper-evaporation or reduced production of lachrymal fluid, the increase of the salt concentration increases the osmolarity of the film tear, with negative consequences on the ocular surface.

According to some preferred embodiments of the invention, therefore, the proposed topical ophthalmic preparation also includes one a more agents of one or more of the following categories: regulators of tonicity agents, pH regulating agents, preservatives or antimicrobial agents.

As regards the production of the preparations of the invention procedure, it is known that the sequence of additions of the various excipients and the active ingredients during the preparation of a hydrogel can affect some of the physical and chemical characteristics of the same formulation, such as viscosity, particle size, degree of dispersion of the active ingredients as well as homogeneity of the system. The preferred operating procedure is schematized in general terms as follows:
- dissolution of one or more osmotizing agents and the one or more polymeric gelling in water;
- possible addition, of polymeric emulsifier under stirring;
- addition of the mixture of polyunsaturated fatty acids already mixed with the oily vehicle;
- addition of an aqueous solution with the buffer and any other salts;
- gelation by the addition of NaOH.

Alternatively, the amount of NaOH required for gelation can be mixed beforehand with the salt solution containing the buffer, and the resulting solution may be added to the oil dispersion in the previously obtained polymer solution.

All additions must be performed under mechanical stirring, preferably at 200 rpm. From this preparation procedure, a distribution of the radius of the oil-phase domains between 1 µm and 5 µm was obtained, with an average value of about 3 µm.

The specific features of the invention, as well as the advantages thereof, will become more apparent with reference to the detailed description presented merely by way of example in the following, together with the results of experiments carried out on it and the data comparison with the prior art. Some experimental results are also illustrated in the accompanying drawings, wherein:
**Figures 1a** and **1b** show, in diagram form, the results of a study of the chemical stability of a preparation according to Example 3 of the patent application WO2010/106571 (formulation MDV0705), stored at 4°C and 25°C for 24 months , respectively in terms of the EPA content of and in terms of DHA content;
**Figures 2a** and **2b** show, in diagram form, the results of a study of the chemical stability of a preparation according to Example 3 of the patent application WO2010/106571 (formulation MDV0705) in comparison with the preparation from Example 6 of the present invention (formulation FHP-NEW) both stored at 25°C for 24 months, respectively in terms of the EPA content and the DHA content;
**Figure 3** shows the tear secretion measured in terms of break-up time (BUT) at day 0 and day 7 in patients treated with a preparation in hydrogel according to the invention (FHP-NEW) in comparison with the prior art formulation MDV0705; and
**Figure 4** shows the tear secretion measured with Schirmer The test on day 0 and day 7 in patients treated with the preparation in hydrogel according to the invention (FHP-NEW) in comparison with the prior art formulation MDV0705 .

### EXAMPLES 1-5

### Compositions with MCT and carbopol

Some preparations according to the invention have been obtained by the following procedure:
- the osmotizing agent was weighed into an appropriate container, and then it was diluted in water;
- the polymers according to the formulation Examples were added to the previous solution at room temperature, adding later, when required, the polymers with emulsifying properties, and the mixture was kept under stirring at 200 rpm until complete dissolution of the polymers;
- in a separate container, the oily phase consisting of the PUFAs in admixture with triglycerides, etc. was prepared;
- in a third container, a basic aqueous solution of NaOH at 4% was prepared and the desired buffer and EDTA, when present, were added therein and the whole admixture was kept under stirring until complete dissolution of the salts;
   the oily phase was added to the solution containing the polymer at room temperature and under vigorous stirring, and the dispersion was maintained under stirring for 30 min;
- finally, the previously prepared basic solution was added to the oil/polymer solution dispersion, maintaining a vigorous stirring for at least 30 minutes.

The products resulting from the described procedure resulted to be stable dispersions of oil globules having the average size of a few micrometers containing the active ingredients, in an aqueous hydrogel matrix.

| **Composition** | **Example 1** | **Example 2** | **Example 3** | **Example 4** | **Example 5** |
|---|---|---|---|---|---|
| | **w/w%** | | | | |
| EPA EE | 0.05 | 0.05 | 0.15 | 0.05 | 0.01 |
| DHA EE | 0.01 | 0.01 | 0.015 | 0.01 | 0.03 |
| **MCT** | **0.25** | **0.40** | **0.60** | **0.25** | **0.20** |

| Aqueous phase | | | | | |
|---|---|---|---|---|---|
| Carbopol | 0.20 | 0.20 | 0.20 | 0.20 | 0.20 |
| Pemulen tr-1 | 0.01 | 0.01 | 0.01 | 0.01 | 0.01 |
| Glycerol | 0.70 | 0.70 | 0.90 | 0.70 | 0.70 |
| Trehalose | 3.00 | 3.00 | 2.00 | 3.00 | 3.00 |
| Na₂HPO₄∗12H₂O | - | - | - | - | 0.25 |
| Sodium citrate | 0.10 | 0.10 | 0.10 | - | - |
| EDTA-Na₂∗2H₂O | 0.02 | 0.02 | 0.02 | 0.02 | 0.02 |
| NaOH | to pH 7 | to p pH 7 | to pH 7 | to pH 7 | to pH 7 |
| Water for injection | to 100 g | to 100 g | to 100 g | to 100 g | to 100 g |

### Examples 6-9

### Composition with MCT, carbopol and hyaluronic acid

The following preparations were obtained by the same operation procedure of Examples 1-5.

| **Composition** | **Example 6** | **Example 7** | **Example 8** | **Example 9** |
|---|---|---|---|---|
| | **w/w%** | | | |
| EPA EE | 0.01 | 0.01 | 0.10 | 0.01 |
| DHA EE | 0.03 | 0.03 | 0.05 | 0.03 |
| **MCT** | **0.20** | **0.25** | **0.15** | **0.20** |

| Aqueous phase | | | | |
|---|---|---|---|---|
| Carbopol | 0.20 | 0.20 | 0.10 | 0.20 |
| Hyaluronic acid | 0.075 | 0.075 | 0.05 | 0.075 |

| | | | | |
|---|---|---|---|---|
| Pemulen tr-1 | 0.01 | 0.01 | 0.01 | - |
| Sodium caproyl hyaluronate | - | - | - | 0.02 |
| Glycerol | 0.70 | 0.70 | - | 0.70 |
| Trehalose | 3.00 | 3.00 | 4,00 | 3.00 |
| Na₂HPO₄∗12H₂O | - | - | 0.10 | 0.25 |
| Sodium citrate | 0.10 | 0.05 | - | - |
| EDTA-Na₂∗2H₂O | 0.02 | 0.02 | 0.01 | 0.02 |
| NaOH | to pH 7 | to pH 7 | to pH 7 | to pH 7 |
| Water for injection | to 100 g | to 100 g | to 100 g | to 100 g |

### Examples 10-13

### Preparations with MCT and other gelling polymers

The following preparations were obtained by the same operation procedure of the previous examples.

| **Composition** | **Example 10** | **Example 11** | **Example 12** | **Example 13** |
|---|---|---|---|---|
| | **w/w%** | | | |
| EPA EE | 0.15 | 0.15 | 0.10 | 0.10 |
| DHA EE | 0.01 | 0.15 | 0.05 | 0.05 |
| **MCT** | **0.35** | **0.40** | **0.25** | **0.35** |

| Aqueous phase | | | | |
|---|---|---|---|---|
| Hydroxypropyl methylcellulose | 0.50 | - | - | - |
| Carboxymethylcellulose | - | 0.40 | - | - |
| Polycarbophyl | - | - | 0.15 | 0.15 |
| Hyaluronic acid | - | - | 0.25 | 0.25 |
| Pemulen tr-1 | 0.01 | 0.01 | 0.01 | 0.01 |
| Sodium caproyl hyaluronate | - | - | 0.05 | 0.05 |
| TPGS | - | - | 0.01 | 0.01 |
| Glycerol | 0.70 | 0.90 | - | - |

| | | | | |
|---|---|---|---|---|
| Trehalose | 3.00 | 2,00 | 4,00 | 4,00 |
| Na₂HPO₄∗12H₂O | 0.25 | - | - | - |
| Sodium borate | - | 0.10 | - | - |
| Sodium citrate | - | - | 0.05 | 0.05 |
| EDTA-Na₂∗2H₂O | 0.02 | - | 0.01 | - |
| NaOH | to pH 7 | to pH 7 | to pH 7 | to pH 7 |
| Water for injection | to 100 g | to 100 g | to 100 g | to 100 g |

### Examples 14-17

### Preparations with coconut oil and carbopol

The following preparations, wherein the carrier of the oil phase is constituted by coconut oil, have been obtained by the same operation procedure of the previous examples.

| **Composition** | **Example 14** | **Example 15** | **Example 16** | **Example 17** |
|---|---|---|---|---|
| | **w/w%** | | | |
| EPA EE | 0.05 | 0.10 | 0.01 | 0.40 |
| DHA EE | 0.01 | 0.40 | 0.03 | 0.40 |
| **Coconut oil** | **0.25** | **0.50** | **0.20** | **0.70** |

| Aqueous phase | | | | |
|---|---|---|---|---|
| Carbopol | 0.20 | 0.20 | 0.20 | 0.20 |
| Hyaluronic acid | | | 0.075 | |
| Pemulen tr-1 | 0.01 | 0.01 | 0.01 | 0.01 |
| Glycerol | 0.70 | 1,15 | 0.70 | - |
| Trehalose | 3.00 | - | 3.00 | 3.00 |
| Na₂HPO₄∗12H₂O | 0.25 | 0.25 | 0.25 | 0.25 |
| EDTA-Na₂∗2H₂O | 0.02 | 0.02 | 0.02 | 0.02 |
| NaOH | to pH 7 | to pH 7 | to pH 7 | to pH 7 |
| Water for injection | to 100 g | to 100 g | to 100 g | to 100 g |

### Examples 18-21

### Preparations with castor oil and carbopol

The following preparations, wherein the carrier of the oily phase consists of castor oil, were obtained by the same operation procedure of the previous examples.

| **Composition** | **Example 17** | **Example 18** | **Example 19** | **Example 20** | **Example 21** |
|---|---|---|---|---|---|
| | **w/w%** | | | | |
| EPA EE | 0.20 | 0.075 | 0.075 | 0.30 | 0.10 |
| DHA EE | 0.10 | 0.075 | 0.075 | 0.20 | 0.05 |
| **Castor oil** | **0.50** | **0.30** | **0.30** | **0.60** | **0.40** |

| Aqueous phase | | | | | |
|---|---|---|---|---|---|
| Carbopol | 0.20 | 0.20 | 0.20 | 0.30 | 0.10 |
| Hydroxypropyl guar | - | - | 0.10 | - | - |
| Hyaluronic acid | - | - | - | - | 0.25 |
| Pemulen tr-1 | 0.01 | 0.01 | 0.01 | 0.01 | 0.01 |
| TPGS | - | - | - | 0.01 | - |
| Glycerol | 0.70 | 1,15 | 1,15 | 1,15 | - |
| Trehalose | 3.00 | 0.50 | - | - | 4,0 |
| Na₂HPO₄∗12H₂O | 0.25 | 0.25 | 0.25 | - | - |
| Sodium citrate | - | - | - | 0.10 | 0.05 |
| EDTA-Na₂∗2H₂O | 0.02 | 0.02 | 0.02 | 0.04 | 0.01 |
| NaOH | to pH 7 | to pH 7 | to pH 7 | to pH 7 | to pH 7 |
| Water for injection | to 100 g | to 100 g | to 100 g | to 100 g | to 100 g |

### Examples 22-26

### Preparations with castor oil and other gelling polymers

The following preparations, wherein the carrier of the oily phase consists of castor oil and carbopol is substituted by other gelling polymers, were obtained by the same operation procedure of the previous examples.

| **Composition** | **Example 22** | **Example 23** | **Example 24** | **Example 25** | **Example 26** |
|---|---|---|---|---|---|
| | **w/w%** | | | | |
| EPA EE | 0.075 | 0.075 | 0.15 | 0.05 | 0.15 |
| DHA EE | 0.075 | 0.075 | 0.15 | 0.15 | 0.15 |
| **Castor oil** | **0.30** | **0.30** | **0.30** | **0.30** | **0.30** |

| Aqueous phase | | | | | |
|---|---|---|---|---|---|
| Carbopol | - | - | - | - | 0.05 |
| Hydroxypropyl guar | 0.10 | - | - | - | - |
| Xanthan gum | - | 0.25 | - | - | - |
| Hydroxypropyl methylcellulose | - | - | - | 0.25 | - |
| Carboxymethylcellulose | - | - | 0.40 | - | 0.40 |
| Hyaluronic acid | 0.25 | - | - | 0.15 | - |
| Pemulen tr-1 | 0.01 | 0.05 | 0.10 | 0.10 | 0.10 |
| Sodium caproyl hyaluronate | - | - | - | 0.02 | - |
| TPGS | - | - | - | - | 0.02 |
| Glycerol | 0.90 | - | 0.90 | 0.70 | 0.90 |
| Trehalose | 2,00 | 4,0 | 2,00 | 3.00 | 2,00 |
| Na₂HPO₄∗12H₂O | 0.25 | 0.25 | - | - | - |
| Sodium citrate | - | - | - | - | - |
| Sodium borate | - | - | 0.1 | 0.05 | 0.10 |
| EDTA-Na₂∗2H₂O | 0.02 | 0.02 | - | 0.03 | 0.01 |
| NaOH | to pH 7 | to pH 7 | to pH 7 | to pH 7 | to pH 7 |
| Water for injection | to 100 g | to 100 g | to 100 g | to 100 g | to 100 |

### Examples 27-29

### Preparations with soybean oil

The following preparations, wherein the carrier of the oily phase consists of soybean oil, were obtained by the same operation procedure of the previous examples.

| **Composition** | **Example 27** | **Example 28** | **Example 29** |
|---|---|---|---|
| | **w/w%** | | |
| EPA EE | 0.30 | 0.05 | 0.03 |
| DHA EE | 0.20 | 0.05 | 0.01 |
| **Soybean oil** | **0.60** | **0.30** | **0.30** |

| Aqueous phase | | | |
|---|---|---|---|
| Carbopol | 0.30 | 0.30 | 0.10 |
| Hyaluronic acid | - | - | 0.15 |
| Pemulen tr-1 | 0.01 | 0.10 | 0.10 |
| Glycerol | 1,15 | 0.90 | 0.70 |
| Trehalose | - | 2,00 | 3.00 |
| Na₂HPO₄∗12H₂O | - | 0.05 | - |
| Sodium citrate | 0.10 | - | - |
| Sodium borate | - | - | 0.05 |
| EDTA-Na₂∗2H₂O | 0.04 | - | 0.03 |
| NaOH | to pH 7 | to pH 7 | to pH 7 |
| Water for injection | to 100 g | to 100 g | to 100 g |

### Examples 30-32

### Preparations with olive oil

The following preparations, wherein the carrier of the oily phase consists of olive oil, were obtained by the same operation procedure of the previous examples.

| **Composition** | **Example 30** | **Example 31** | **Example 32** |
|---|---|---|---|
| | **w/w%** | | |
| EPA EE | 0.05 | 0.10 | 0.05 |
| DHA EE | 0.01 | 0.40 | 0.01 |
| **Olive oil** | **0.25** | **0.50** | **0.25** |

| Aqueous phase | | | |
|---|---|---|---|
| Carbopol | 0.20 | 0.20 | - |
| Hydroxypropyl methylcellulose | - | - | 0.50 |
| Pemulen tr-1 | 0.01 | 0.01 | 0.01 |
| Glycerol | 0.7 | 1,15 | - |
| Trehalose | 3.0 | - | 3.0 |
| Na₂HPO₄∗12H₂O | 0.25 | 0.25 | 0.25 |
| EDTA-Na₂∗2H₂O | 0.02 | 0.02 | 0.02 |
| NaOH | to pH 7 | to pH 7 | to pH 7 |
| Water for injection | to 100 g | to 100 g | to 100 g |

### Examples 33-37

### Preparations with corn oil

The following preparations, wherein the carrier of the oily phase consists of corn oil, were obtained by the same operation procedure of the previous examples.

| **Composition** | **Example 33** | **Example 34** | **Example 35** | **Example 36** | **Example 37** |
|---|---|---|---|---|---|
| | **w/w%** | | | | |
| EPA EE | 0.35 | 0.30 | 0.1 | 0.35 | 0.35 |
| DHA EE | 0.35 | 0.20 | 0.05 | 0.35 | 0.35 |
| **Corn oil** | **0.30** | **0.60** | **0.40** | **0.30** | **0.30** |

| Aqueous phase | | | | | |
|---|---|---|---|---|---|
| Carbopol | 0.20 | 0.20 | - | - | - |
| Polycarbophyl | - | - | 0.15 | - | - |
| Carboxymethylcellulose | - | - | - | 0.40 | - |
| Hydroxypropyl methylcellulose | - | - | - | - | 0.25 |
| Hyaluronic acid | - | - | 0.25 | - | 0.15 |
| Pemulen tr-1 | 0.01 | 0.01 | 0.01 | 0.10 | 0.10 |
| Glycerol | 1,15 | 0.7 | 0.7 | - | 0.9 |
| Trehalose | - | 3.0 | 3.0 | 4,0 | 2,0 |
| Na₂HPO₄∗12H₂O | 0.25 | 0.25 | 0.25 | 0.25 | 0.25 |
| EDTA-Na₂∗2H₂O | 0.02 | 0.02 | 0.02 | 0.02 | 0.02 |
| NaOH | to pH 7 | to pH 7 | to pH 7 | to pH 7 | to pH 7 |
| Water for injection | to 100 g | to 100 g | to 100 g | to 100 g | to 100 g |

### Example 38

### Preparation with sunflower seed oil

The following preparation, wherein the carrier of the oily phase consists of sunflower seed oil, was obtained by the same operation procedure of the previous examples.

| **Composition** | **w/w%** |
|---|---|
| EPA EE | 1,20 |
| DHA EE | 0.80 |
| **Sunflower seed oil** | **2,00** |

| Aqueous phase | |
|---|---|
| Carbopol | 0.2 |
| Pemulen tr-1 | 0.01 |
| Glycerol | 1,15 |
| Na₂HPO₄∗12H₂O | 0.25 |
| EDTA-Na₂∗2H₂O | 0.02 |
| NaOH | to pH 7 |
| Water for injection | to 100 g |

### Examples 39-43

### Presence of an omega-6 (GLA EE) PUFA

The following preparations based on two omega-3 PUFAs and one omega-6 PUFA, GLA, containing as oily carrier MCT or castor oil, were obtained by the same operation procedure of Examples 1-5.

| **Composition** | **Example 39** | **Example 40** | **Example 41** | **Example 42** | **Example 43** |
|---|---|---|---|---|---|
| | **w/w%** | | | | |
| EPA EE | 0.01 | 0.30 | 0.10 | 0.15 | 0.05 |
| DHA EE | 0.03 | 0.20 | 0.05 | 0.15 | 0.15 |
| GLA EE | 0.03 | 0.10 | 0.05 | 0.15 | 0.30 |
| **Castor oil** | **-** | **0.65** | **0.40** | | **0.55** |
| **MCT** | **0.20** | | | **0.55** | |

| Aqueous phase | | | | | |
|---|---|---|---|---|---|
| Carbopol | 0.20 | 0.30 | - | - | - |
| Polycarbophyl | - | - | 0.15 | - | - |
| Carboxymethylcellulose | - | - | - | 0.40 | - |
| Hydroxypropyl methylcellulose | - | - | - | - | 0.25 |
| Hyaluronic acid | 0.075 | - | 0.25 | - | 0.15 |
| Pemulen tr-1 | - | - | - | 0.10 | 0.10 |
| Sodium caproyl hyaluronate | 0.02 | | 0.01 | 0.05 | |
| TPGS | | 0.01 | | 0.02 | 0.03 |
| Glycerol | 0.70 | 1,15 | | 0.9 | 0.70 |
| Trehalose | 3.0 | | 4,0 | 2,0 | 3.0 |
| Na₂HPO₄∗12H₂O | 0.25 | | | | |
| Sodium citrate | | 0.10 | 0.05 | | |
| Sodium borate | | | | 0.1 | 0.05 |
| EDTA-Na₂∗2H₂O | 0.02 | 0.04 | 0.01 | - | 0.03 |
| NaOH | to pH 7 | to pH 7 | to pH 7 | to pH 7 | to pH 7 |
| Water for injection | to 100 g | to 100 g | to 100 g | to 100 g | to 100 g |

### Examples 44-48

### Examples with EPA triglyceride (EPA TG) and DHA triglyceride (DHA TG)

The following preparations based on omega-3 EPA and DHA fatty acids triglycerides and containing as oily carrier MCT, were obtained by the same operation procedure of the Examples 1-5.

| **Composition** | **Example 44** | **Example 45** | **Example 46** | **Example 47** | **Example 48** |
|---|---|---|---|---|---|
| | **w/w%** | | | | |
| EPA TG | 0.06 | 0.06 | 0.25 | 0.1 | 0.2 |
| DHA TG | 0.015 | 0.015 | 0.025 | 0.2 | 0.2 |
| **MCT** | **0.25** | **0.40** | **0.60** | **0.45** | **0.55** |

| Aqueous phase | | | | | |
|---|---|---|---|---|---|
| Carbopol | 0.20 | 0.20 | 0.20 | 0.20 | 0.20 |
| Pemulen tr-1 | 0.01 | 0.01 | 0.01 | 0.01 | 0.01 |
| Glycerol | 0.70 | 0.70 | 0.90 | 0.70 | 0.70 |
| Trehalose | 3.00 | 3.00 | 2,00 | 3.00 | 3.00 |
| Na₂HPO₄∗12H₂O | - | - | - | -0.25 | 0.25 |
| Sodium citrate | 0.10 | 0.10 | 0.10 | - | - |
| EDTA-Na₂∗2H₂O | 0.02 | 0.02 | 0.02 | 0.02 | 0.02 |
| NaOH | to pH 7 | to pH 7 | to pH 7 | to pH 7 | to pH 7 |
| Water for injection | to 100 g | to 100 g | to 100 g | to 100 g | to 100 g |

As shown below, the preparations of the foregoing example formulations showed a degradation at 25°C of EPA and DHA with a linear trend in time, with a value of the percentage of EPA and DHA in any case not less than 75% after 24 months of storage at room temperature.

### Stability studies and comparison with the prior art

The preparations in hydrogel with PUFAs carried in medium or long chain natural or synthetic oils and free from antioxidants based on vitamin E, obtained according to the teachings of the present invention, were compared with the formulations of PUFAs in hydrogel of the prior art, in which the chemical stability of the active ingredients is entrusted to the presence of vitamin E.

As mentioned above, starting from the compositions described in the patent application WO2010/106571 by Medivis it has been shown, with the preliminary experimentation of the present invention, that in the absence of vitamin E acetate, or in the presence of α-tocopherol only, omega-3 and -6 the fatty acids are chemically unstable. In particular, it has been considered, as an example, the composition from Example 3 of MDV705 formulation, which is reported in the prior art document as follows

**Table 1: Composition of Example 3 of WO2010/106571 (MDV0705)**

| Composition oily phase (% by weight | |
|---|---|
| EPA EE | 6,25 |
| DHA EE | 6,25 |
| GLA EE | 12,5 |
| Total PUFA | 25 |
| Vit. E acetate | 75 |

| Overall composition (% wt) | |
|---|---|
| Oily phase (PUFA + vit. E acetate) | 1,001 |
| Carbopol 980 | 0.20 |
| Glycerol | 1,15 |
| Pemulen | 0.007 |
| NaOH | 0.07 |
| Sodium phosphate disodium | 0.10 |
| Purified water | q.b. to 100 |

For the assessment of the chemical stability, the time course of the percentage concentration of the active ingredients in the preparation compared to the initial concentration was determined at storage conditions of the preparation at 25°C. The determinations were carried out by HPLC.

The results in the case of a formulation such as that of Example 3, but devoid of vitamin E acetate are shown in the following Table 2, which shows rapid degradation in time of the three active ingredients.

**Table 2: Stability of the formulation of Example 3 of MDV0705 without vitamin E acetate.**

| **Determinations** | | Omega 3:% **EPA EE** | Omega 3:% **DHA EE** | Omega 6:% **GLA EE** |
|---|---|---|---|---|
| Temperature | Months | % Titre | % Titre | % Titre |
| **25±2°C** RH: 60±5% | 1 | 93 | 89 | 91 |
| | 2 | 87 | 87 | 86 |
| | 3 | 70 | 71 | 70 |
| | 4 | 54 | 52 | 53 |
| | 5 | 30 | 27 | 31 |
| | 6 | 15 | 15 | 12 |

Even steeper is the degradation of the active ingredients when in place of the vitamin E acetate, the formulation from Example 3 of the cited prior patent application contains α-tocopherol, as it is shown in the following table.

**Table 3: Stability of the formulation of Example 3 by MDV0705 with α-tocopherol instead of vitamin E acetate.**

| **Determinations** | | Omega 3:% **EPA EE** | Omega 3:% **DHA EE** | Omega 3:% **GLA EE** |
|---|---|---|---|---|
| Temperature | Months | % Titre | % Titre | % Titre |
| **25±2°C** RH: 60±5% | 1 | 94 | 92 | 94 |
| | 2 | 54 | 52 | 54 |
| | 3 | 25 | 21 | 25 |
| | 4 | abandoned | abandoned | abandoned |

Considering now the formulations with vitamin E acetate described in the cited prior art (MDV0705), it is to remark that they are stable for 12 months. The study reported in the document itself shows that the temperatures of 4°C and 25°C the PUFAs slightly degrade for 12 months, with a linear trend. However, as already noted, the study of stability later extended up to 24 months still shows a linear trend for the formulations stored at 4°C while the stability data obtained at 25°C describes a polynomial trend, that represents a rapid degradation.

In the following **Table 4** the full stability study of the EPA-EE and DHA-EE of the formulation of Example 3 disclosed in the Medivis patent application over 24 months is reported. The graphical representation of the same data is shown in **Figures 1a** and **1b** of the attached drawings.

**Table 4: Stability study of the formulations MDV0705 according to the Medivis patent application at 24 months.**

| **Determination** | | Omega 3:% **EPA EE** | Omega 3:% **DHA EE** |
|---|---|---|---|
| Temperature | Months | %Titre | % Titre |
| **4°C** | 3 | 100 | 100 |
| | 6 | 101 | 101 |
| | 9 | 99 | 99 |
| | 12 | 100 | 100 |
| | 15 | 98 | 98 |
| | 18 | 97 | 97 |
| | 21 | 95 | 95 |
| | 24 | 92 | 90 |

| **Determination** | | Omega 3:% **EPA EE** | Omega 3:% **DHA EE** |
|---|---|---|---|
| Temperature | Months | % Titre | % Titre |
| **25°C** RH: 60±5% | 3 | 100 | 99 |
| | 6 | 100 | 99 |
| | 9 | 97 | 97 |
| | 12 | 95 | 96 |
| | 15 | 89 | 91 |
| | 18 | 70 | 74 |
| | 21 | 50 | 55 |
| | 24 | 25 | 21 |

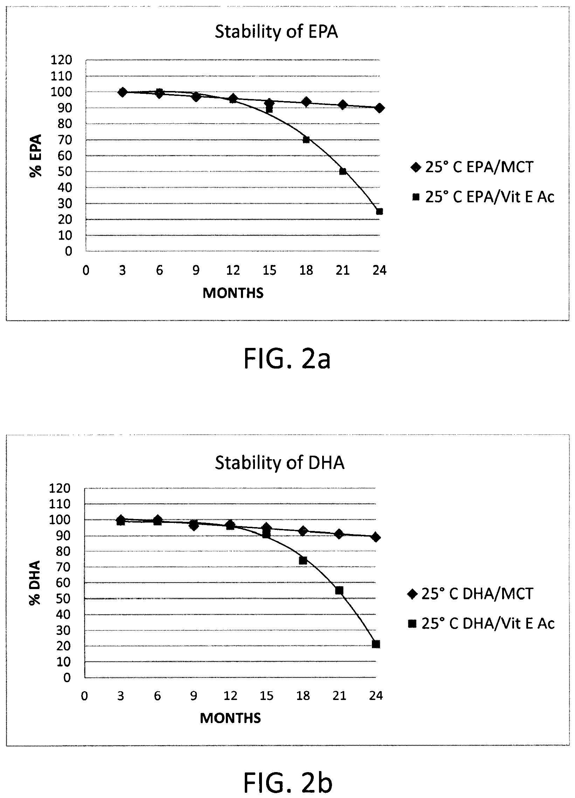

In **Table 5** below, the results of a similar stability study on the preparation described in Example 6 of the present invention are reported, wherein MCTs replace vitamin E acetate. This formulation is referred to below as FHP-NEW formulation. The data of **Table 5** and in **Figures 2a** and **2b** of the attached drawings show the surprising improvement in stability at 25°C of the FHP-NEW formulation containing MCT if compared with the formulation MDV0705 containing vitamin E acetate.

**Table 1: Stability at 24 months of the preparation FHP-NEW at 25°C**

| **Determination** | | Omega 3:% **EPA EE** | Omega 3:% **DHA EE** |
|---|---|---|---|
| Temperature | Months | % Titre | % Titre |
| **25°C** RH: 60±5% | 3 | 100 | 100 |
| | 6 | 99 | 100 |
| | 9 | 97 | 96 |
| | 12 | 96 | 97 |
| | 15 | 93 | 95 |
| | 18 | 94 | 93 |
| | 21 | 92 | 91 |
| | 24 | 90 | 89 |

### Acute ocular tolerance of eve drops according to the invention

The ocular tolerance of eye drops PHP-NEW under review was assessed after three instillations at 2 hours intervals in rabbits' eyes. Two drops of collyrium were instilled in the right eye of each animal for a total of 3 times in the same day at 2 hour intervals. The group of rabbits consisted of 8 animals (4 males and 4 females).

The condition of the ocular tissues was observed according to the Draize test. The examination was conducted after the third instillation on the day of treatment and then 24, 48 and 72 hours after the first instillation, assigning arbitrary scores to various aspects of the conjunctiva of the iris and cornea.

Any significant reddening of the conjunctiva for the entire period of the test was observed, both in the eyes treated with FHP-NEW and in the eyes treated with placebo. No edema or opacity at the corneal level was detected Moreover, no involvement of the iris was not noticed. The presence of drain material was maintained at the normal level. The results obtained show that the ophthalmic preparation according to the invention is well tolerated after repeated instillation (three in 6 hours), and that there were no differences in comparison with placebo.

### Clinical tolerability tests

The instillation of PHP-NEW eye-drops three times a day on a group of 20 normal subjects, i.e. without any sign of ocular surface pain and with a normal tear secretion, did not produce any significant undesirable effects. In particular, subjects who received FHP-NEW were given a questionnaire with two questions to which they had to answer after 1, 5, 10 and 60 minutes from administration. To the question 1 ("Do you feel stinging after instillation of the eye drops?"), 100% of the subjects answered "no" at each control; to the question 2 ("Do you feel discomfort after instillation of the eye drops?"), 100% of the subjects answered "no" at each control.

### Evaluation of the effectiveness of the preparation according to the invention in the treatment of dry eye syndrome

Given that the preparation PHP-NEW containing omega-3 fatty acids showed to be able to have protective characteristics on the ocular surface and to reduce inflammation, once the tolerability was assessed, its effectiveness in the treatment of patients suffering from dry eye syndrome was assessed.

The product was compared with the eyedrops MDV0705 (Example 3 of International Publication WO2010/106571). The FHP-NEW efficacy was assessed on a group of patients (n = 5) with dry eye syndrome. As a control group (n = 5) patients were used with dry eye syndrome treated with eye drops MDV0705. The patients were instilled FHP-NEW and MDV0705 three times daily in both eyes and checks were carried out after seven days of treatment. The right eye was used for statistical analysis of the results.

Patients with dry eye syndrome were selected following the inclusion criteria that are internationally recognized (The definition and classification of dry eye disease. Subcommittee of the International Dry Eye Workshop, Ocul. Surf. 2007; 5:75-92), and more precisely:
- dry eye symptoms, measured using a questionnaire, with a codified system for the scoring (Shiffman RM, Dale Christianson M, Jacobsen G, Hirsch JD, Reis BL. Reliability and validity of the Ocular Surface Disease Index (OSDI), Arch. Ophthalmol. 2000; 118:615-21;
- fluorescein staining of the cornea measured according to NEI/Industry workshop Scale > 3 (Lemp MA, Report of the National Eye Institute/Industry workshop on clinical trials in dry eyes. CLAO J. 1995; 21:221-232);
- break-up time (BUT) < 10 seconds;
- Schirmer I test< 8 mm at 5 minutes.
- Exclusion criteria: infectious keratoconjunctivitis, a history of ocular allergies, ocular or eyelid surgery in the three months preceding the study, nasolacrimal diseases, use of eye drops steroid in the four weeks preceding the study, anti-glaucoma eye drops and in case of diabetes.

The parameters used to evaluate the effectiveness of FHP-NEW compared to the control were as follows:
- Symptoms of dry eye (measured by questionnaire);
- Tear film break up time or *break-up time* (BUT);
- Schirmer Test I.

### Break-Up-Time (BUT) evaluation procedure

The tear break-up time is considered an indicator of the stability of the tear film. The test consists in observing with the slit lamp with a cobalt blue filter the surface of the film after instillation of fluorescein. During the test, the patient keeps his eyes open without blinking and looking straight ahead, and the time elapsed between the last blink and the formation of small dry areas (which appear darker) on the corneal surface is measured, then computing the average of three successive determinations.

### Schirmer I Test

The Schirmer test I provides information on the tear secretion. It is carried out in a dimly lit room, placing a filter paper strip in the lower conjunctival the fornix at the external corner, and the impregnation of the paper is cheked after 5 minutes. The test should be conducted with standardized procedure as there are many variables involved in determining the value. The most important variable comes from the fact that the absorption by the filter paper strip and the length of the wet portion are influenced by the strength of the capillarity and wettability of the cellulose fibers, hence it appears important to perform the test with validated maps .

### Experimental results of BUT and Schirmer I tests carried out on FHP-NEW vs MDV0705

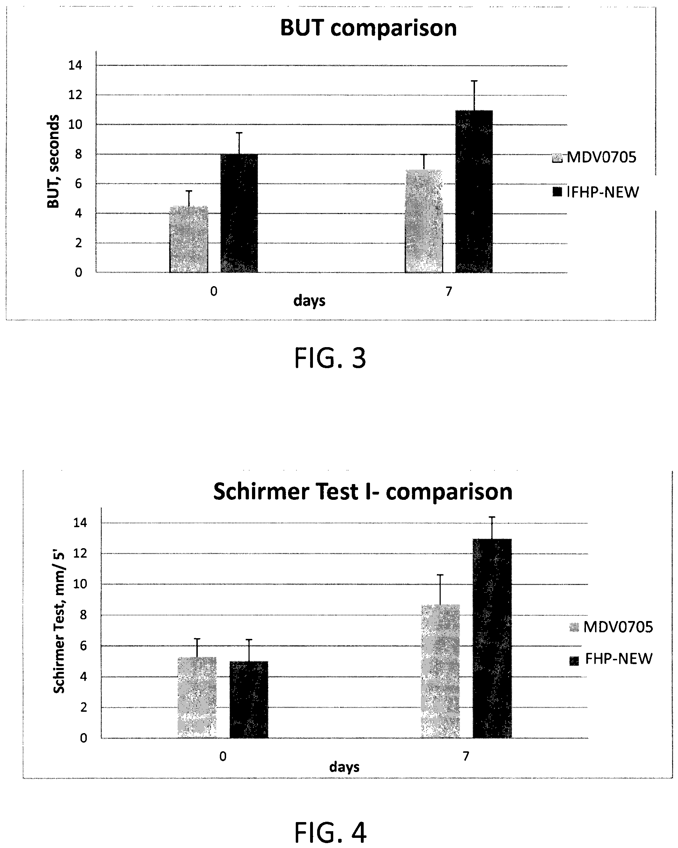

The results of the observational study carried out for the BUT and the Schirmer I test are respectively shown in Figures 3 and 4 of the accompanying drawings.

The results on the seventh day showed a significant improvement of the symptoms and signs of ocular surface in patients with dry eye syndrome treated with FHP-NEW from baseline values (day 0). Moreover, on the seventh day the patients treated with FHP-NEW showed a significant (p <0.05) improvement of the symptoms compared to the control group.

In particular, the tear break time (BUT) on the seventh day showed a significant increase from the baseline (day 0) only in the group of patients treated with FHP-NEW (Figure 3).

The tear secretion measured by the Schirmer test I showed a significant increase compared to the initial conditions only in the group of patients treated with FHP-NEW, while the group of patients treated with MDV0705 showed a non-significant increase in tear production at seven days. The comparison at the seventh day between patients treated with FHP-NEW and patients treated with MDV0705 demonstrated a significant increase in the first group with respect to the second group (Figure 4).

Summarizing, it can be observed that the possibility of using omega-3 and omega-6 fatty acids in eye drops is an important innovation for the treatment of patients suffering from dry eye syndrome, as it both reduces the symptoms complained of by patients, and improves the stability of the tear film and, accordingly, the conditions of the eye surface. Such beneficial therapeutic tool is easily available from the pharmaceutical point of view, thanks to the improved shelf-life obtainable with the formulations according to the invention.

## Claims

1. A topical ophthalmic preparation containing, as active ingredients, one or more polyunsaturated fatty acids selected from the group consisting of: omega-3 polyunsaturated fatty acids and omega-6 polyunsaturated fatty acids, said polyunsaturated fatty acids having an aliphatic chain of from 16 to 24 carbon atoms, or pharmaceutically acceptable derivatives thereof selected from their esters with C₁-C₄ alkyl groups and their triglycerides, in solution in an oily vehicle,
said oily vehicle consisting of a vegetable or synthetic oil selected from the group consisting of: medium chain triglycerides (MCT), long chain triglycerides (LCT) liquid at room temperature, vegetable oils and their mixtures, and not containing vitamin E or pharmaceutically acceptable esters thereof,
said solution being dispersed in form of oil globules in a hydrogel based on an aqueous vehicle containing one or more gelling polymers,
**characterised in that** said oil globules have an average diameter of 1-10 µm, and **in that** said preparation contains an overall amount of not more than 0.10% by weight of surfactants and emulsifying agents, and is substantially stable at room temperature, having a remaining content of undegraded active ingredients of not less than 90% by weight of the starting content after 18 months from the date of manufacture, when stored at 25°C.

2. A topical ophthalmic preparation according to claim 1, wherein said one or more polyunsaturated fatty acids are in solution in said oily vehicle at a concentration of from 5% to 85% by weight.

3. A topical ophthalmic preparation according to claims 1 or 2, wherein said omega-3 and omega-6 fatty acids have aliphatic chains of from 18 to 22 carbon atoms.

4. A topical ophthalmic preparation according to claim 3, wherein said oil globules have an average diameter of 1-5 µm.

5. A topical ophthalmic preparation according to claim 3, wherein said surfactants and emulsifying agents are present in an overall amount of no more than 0.010% by weight.

6. A topical ophthalmic preparation according to claim 5, wherein said surfactants and emulsifiers are polymeric emulsifying agents selected from the group consisting of: high molecular weight crosslinked polyacrylates, polyoxyethylene-polyoxypropylene block copolymers, D-alpha-tocopheryl polyethylene glycol succinate.

7. A topical ophthalmic preparation according to claim 3, wherein said oily vehicle consists of medium chain triglycerides (MCT).

8. A topical ophthalmic preparation according to claim 3, wherein said vegetable oil is selected from the group consisting of: coconut oil, castor oil, soybean oil, olive oil, corn oil, sunflower seed oil, sesame oil and mixtures thereof.

9. A topical ophthalmic preparation according to claim 3 containing, as omega-3 polyunsaturated fatty acid, eicosapentaenoic acid (EPA) or a linear or branched C₁-C₄ alkyl ester or the triglyceride thereof, and/or docosahexaenoic acid (DHA) or a linear or branched C1-C₄ alkyl ester or the triglyceride thereof, and, as omega-6 polyunsaturated fatty acid, γ-linolenic acid (GLA) or a linear or branched C₁-C₄ alkyl ester thereof or the triglyceride thereof, or mixtures of the same.

10. A topical ophthalmic preparation according to claim 9, containing, as active ingredients, EPA, DHA and optionally GLA, or the ethyl esters thereof.

11. A topical ophthalmic preparation according to claim 10, wherein the concentrations of EPA and DHA are each from 0.001% to 1.2% by weight, and the concentration of said oily vehicle is from 0.005% to 2% by weight, the remainder being made of the said hydrogel.

12. A topical ophthalmic preparation according to claim 3, wherein said one or more gelling polymers are selected from: carboxyvinyl polymers, hyaluronic acid and the alkali or alkali-earth metal salts thereof, cellulose esters and ethers, xanthan gum, gellans, alginic acid and alginates, and mixtures thereof.

13. A topical ophthalmic preparation according to claim 7, wherein said gelling polymer is carbopol.

14. A topical ophthalmic preparation according to claim 3, further comprising one or more agents of the following categories: tonicity adjusting agents, pH adjusting agents, preservatives or antimicrobials.

## Patentansprüche

1. Topische ophthalmische Zubereitung, enthaltend als Wirkstoffe eine oder mehrere mehrfach ungesättigte Fettsäuren, ausgewählt aus der Gruppe, bestehend aus: mehrfach ungesättigten Omega-3-Fettsäuren und mehrfach ungesättigten Omega-6-Fettsäuren, wobei die mehrfach ungesättigten Fettsäuren eine aliphatische Kette von 16 bis 24 Kohlenstoffatomen aufweisen, oder pharmazeutisch verträgliche Derivate davon, ausgewählt aus ihren Estern mit C₁-C₄-Alkylgruppen und ihren Triglyceriden, in Lösung in einem öligen Träger,
wobei der ölige Träger aus einem pflanzlichen oder synthetischen Öl besteht, ausgewählt aus der Gruppe, bestehend aus: mittelkettigen Triglyceriden (MCT), langkettigen Triglyceriden (LCT), die bei Raumtemperatur flüssig sind, pflanzlichen Ölen und deren Gemischen, und kein Vitamin E oder pharmazeutisch verträgliche Ester davon enthält,
wobei die Lösung in Form von Ölkügelchen in einem Hydrogel auf der Basis eines wässrigen Trägers dispergiert ist, der ein oder mehrere gelierende Polymere enthält,
**dadurch gekennzeichnet, dass** die Ölkügelchen einen mittleren Durchmesser von 1-10 µm aufweisen, und dass die Zubereitung eine Gesamtmenge von nicht mehr als 0,10 Gewichts-% Tenside und Emulgatoren enthält,
und bei Raumtemperatur im Wesentlichen stabil ist, mit einem Restgehalt an nicht abgebauten Wirkstoffen von nicht weniger als 90 Gewichts-% des Ausgangsgehalts nach 18 Monaten ab Herstellungsdatum bei Lagerung bei 25 °C.

2. Topische ophthalmische Zubereitung nach Anspruch 1, wobei die eine oder die mehreren mehrfach ungesättigten Fettsäuren in Lösung in dem öligen Träger in einer Konzentration von 5 bis 85 Gewichts-% vorliegen.

3. Topische ophthalmische Zubereitung nach Ansprüchen 1 oder 2, wobei die Omega-3- und Omega-6-Fettsäuren aliphatische Ketten mit 18 bis 22 Kohlenstoffatomen aufweisen.

4. Topische ophthalmische Zubereitung nach Anspruch 3, wobei die Ölkügelchen einen mittleren Durchmesser von 1-5 µm aufweisen.

5. Topische ophthalmische Zubereitung nach Anspruch 3, wobei die Tenside und Emulgatoren in einer Gesamtmenge von nicht mehr als 0,010 Gewichts-% vorhanden sind.

6. Topische ophthalmische Zubereitung nach Anspruch 5, wobei die Tenside und Emulgatoren polymere Emulgatoren sind, ausgewählt aus der Gruppe, bestehend aus: hochmolekularen vernetzten Polyacrylaten, Polyoxyethylen-Polyoxypropylen-Blockcopolymeren, D-alpha-Tocopherylpolyethylenglykolsuccinat.

7. Topische ophthalmische Zubereitung nach Anspruch 3, wobei der ölige Träger aus mittelkettigen Triglyceriden (MCT) besteht.

8. Topische ophthalmische Zubereitung nach Anspruch 3, wobei das Pflanzenöl ausgewählt ist aus der Gruppe, bestehend aus: Kokosöl, Rizinusöl, Sojaöl, Olivenöl, Maisöl, Sonnenblumenkernöl, Sesamöl und Gemischen davon.

9. Topische ophthalmische Zubereitung nach Anspruch 3, enthaltend als mehrfach ungesättigte Omega-3-Fettsäure Eicosapentaensäure (EPA) oder einen linearen oder verzweigten C₁-C₄-Alkylester oder das Triglycerid davon, und/oder Docosahexaensäure (DHA) oder einen linearen oder verzweigten C₁-C₄-Alkylester oder das Triglycerid davon und als mehrfach ungesättigte Omega-6-Fettsäure γ-Linolensäure (GLA) oder einen linearen oder verzweigten C₁-C₄-Alkylester davon oder das Triglycerid davon oder Gemische davon.

10. Topische ophthalmische Zubereitung nach Anspruch 9, enthaltend als Wirkstoffe EPA, DHA und gegebenenfalls GLA oder die Ethylester davon.

11. Topische ophthalmische Zubereitung nach Anspruch 10, wobei die Konzentrationen von EPA und DHA jeweils 0,001 bis 1,2 Gewichts-% betragen und die Konzentration des öligen Trägers 0,005 bis 2 Gewichts-% beträgt, wobei der Rest aus dem Hydrogel besteht.

12. Topische ophthalmische Zubereitung nach Anspruch 3, wobei das eine oder die mehreren gelierenden Polymere ausgewählt sind aus: Carboxyvinylpolymeren, Hyaluronsäure und den Alkali- oder Erdalkalimetallsalzen davon, Celluloseestern und - ethern, Xanthangummi, Gellanen, Alginsäure und Alginaten und Gemischen davon.

13. Topische ophthalmische Zubereitung nach Anspruch 7, wobei das gelierende Polymer Carbopol ist.

14. Topische ophthalmische Zubereitung nach Anspruch 3, weiter umfassend ein oder mehrere Mittel der folgenden Kategorien: tonizitätseinstellende Mittel, pH-Einstellmittel, Konservierungsmittel oder antimikrobielle Mittel.

## Revendications

1. Préparation ophtalmique topique contenant, en tant que principes actifs, un ou plusieurs acides gras polyinsaturés sélectionnés à partir du groupe constitué par : les acides gras polyinsaturés oméga-3 et les acides gras polyinsaturés oméga-6, lesdits acides gras polyinsaturés ayant une chaîne aliphatique de 16 à 24 atomes de carbone, ou dérivés pharmaceutiquement acceptables de ceux-ci, sélectionnés à partir de leurs esters avec des groupes alkyle en C₁-C₄ et leurs triglycérides, en solution dans un véhicule huileux,
ledit véhicule huileux constitué d'une huile végétale ou synthétique sélectionnée à partir du groupe constitué par : les triglycérides à chaîne moyenne (MCT), les triglycérides à chaîne longue (LCT) liquides à température ambiante, les huiles végétales et leurs mélanges, et ne contenant pas de vitamine E ou les esters pharmaceutiquement acceptables ceux-ci,
ladite solution étant dispersée sous forme de globules d'huile dans un hydrogel à base d'un véhicule aqueux contenant un ou plusieurs polymères gélifiants,
**caractérisé en ce que** lesdits globules d'huile ont un diamètre moyen de 1 à 10 µm, et **en ce que** ladite préparation contient une quantité globale ne dépassant pas 0,10% en poids de tensioactifs et d'agents émulsifiants,
et est substantiellement stable à température ambiante, ayant une teneur restante en ingrédients actifs non dégradés de pas moins de 90% en poids de la teneur de départ après 18 mois à partir de la date de fabrication, quand stocké à 25 ° C.

2. Préparation ophtalmique topique selon la revendication 1, dans laquelle ledit ou lesdits acides gras polyinsaturés sont en solution dans ledit véhicule huileux à une concentration de 5% à 85% en poids.

3. Préparation ophtalmique topique selon les revendications 1 ou 2, dans laquelle lesdits acides gras oméga-3 et oméga-6 ont des chaînes aliphatiques de 18 à 22 atomes de carbone.

4. Préparation ophtalmique topique selon la revendication 3, dans laquelle lesdits globules d'huile ont un diamètre moyen de 1 à 5 µm.

5. Préparation ophtalmique topique selon la revendication 3, dans laquelle lesdits agents tensioactifs et agents émulsifiants sont présents en une quantité globale ne dépassant pas 0,010% en poids.

6. Préparation ophtalmique topique selon la revendication 5, dans laquelle lesdits agents tensioactifs et émulsifiants sont des agents émulsifiants polymères sélectionnés à partir du groupe constitué par : les polyacrylates réticulés de poids moléculaire élevé, les copolymères séquencés de polyoxyéthylène-polyoxypropylène, du succinate de polyéthylèneglycol de D-alpha-tocophéryle.

7. Préparation ophtalmique topique selon la revendication 3, dans laquelle ledit véhicule huileux est constitué de triglycérides à chaîne moyenne (MCT).

8. Préparation ophtalmique topique selon la revendication 3, dans laquelle ladite huile végétale est sélectionnée à partir du groupe constitué par : l'huile de coco, l'huile de ricin, l'huile de soja, l'huile d'olive, l'huile de maïs, l'huile de graines de tournesol, l'huile de sésame et les mélanges de ceux-ci.

9. Préparation ophtalmique topique selon la revendication 3 contenant, comme acide gras polyinsaturé oméga-3, l'acide eicosapentaénoïque (EPA) ou un ester d'alkyle en C₁-C₄ linéaire ou ramifié ou le triglycéride de ceux-ci, et/ou l'acide docosahexaénoïque (DHA) ou un ester d'alkyle en C₁-C₄ linéaire ou ramifié ou le triglycéride de ceux-ci, et, en tant qu'acide gras polyinsaturé oméga-6, l'acide γ-linolénique (GLA) ou un ester d'alkyle en C₁-C₄ linéaire ou ramifié ou le triglycéride de ceux-ci, ou mélanges de ceux-ci.

10. Préparation ophtalmique topique selon la revendication 9, contenant en tant que principes actifs l'EPA, le DHA et optionnellement le GLA, ou les esters éthyliques de ceux-ci.

11. Préparation ophtalmique topique selon la revendication 10, dans laquelle les concentrations d'EPA et de DHA sont chacune de 0,001% à 1,2% en poids, et la concentration dudit véhicule huileux est de 0,005% à 2% en poids, le reste étant constitué dudit hydrogel.

12. Préparation ophtalmique topique selon la revendication 3, dans laquelle ledit ou lesdits polymères gélifiants sont sélectionnés parmi : les polymères carboxyvinyliques, l'acide hyaluronique et les sels de métaux alcalins ou alcalino-terreux de ceux-ci, les esters et éthers de cellulose, la gomme de xanthane, les gélanes, l'acide alginique et les alginates, et les mélanges de ceux-ci.

13. Préparation ophtalmique topique selon la revendication 7, dans laquelle ledit polymère gélifiant est le carbopol.

14. Préparation ophtalmique topique selon la revendication 3, comprenant en outre un ou plusieurs agents des catégories suivantes : agents d'ajustement de la tonicité, agents d'ajustement du pH, conservateurs ou antimicrobiens.
